# EUROPÄISCHE PATENTSCHRIFT

(11) **EP 0 744 469 B1**
(45) Veröffentlichungstag und Bekanntmachung des Hinweises auf die Patenterteilung: **13.11.2002**
(21) Anmeldenummer: 96107481.2
(22) Anmeldetag: 10.05.1996
(51) Int. Cl.: C12Q 1/68

(54) **Inter-LINE-PCR**
Inter LINE (long interspersed elements) PCR
PCR inter LINE

(30) Priorität: 22.05.1995 DE 19518769
(43) Veröffentlichungstag der Anmeldung: 27.11.1996
(73) Patentinhaber: GSF-Forschungszentrum für Umwelt und Gesundheit GmbH, 85764 Oberschleissheim (DE)
(72) Erfinder: Smida, Jan Dr., 85354 Freising (DE); Leibhard, Stefan, 85764 Oberschleissheim (DE); Hieber, Ludwig, Dr., 85551 Kirchheim (DE); Eckardt-Schupp, F. Prof. Dr., 85276 Pfaffenhofen (DE)
(74) Vertreter: Reinhard - Skuhra - Weise & Partner

(56) Entgegenhaltungen:
- WO-A-92/01059
- LEDBETTER S A ET AL: 'RAPID ISOLATION OF DNA PROBES WITHIN SPECIFIC CHROMOSOME REGIONS BY INTERSPERSED REPETITIVE SEQUENCE POLYMERASE CHAIN REACTION' GENOMICS Bd. 6, 01 Januar 1990, Seiten 475 - 481, XP000283124 ISSN: 0888-7543
- NELSON D L ET AL: "ALU POLYMERASE CHAIN REACTION: A METHOD FOR RAPID ISOLATION OF HUMAN-SPECIFIC SEQUENCES FROM COMPLEX DNA SOURCES" PROCEEDINGS OF THE NATIONAL ACADEMY OF SCIENCES OF USA, Bd. 86, Nr. 17, 1. September 1989 (1989-09-01), Seiten 6686-6690, XP000310571 ISSN: 0027-8424
- LICHTER P ET AL: "FLUORESCENCE IN SITU HYBRIDIZATION WITH ALU AND L1 POLYMERASE CHAIN REACTION PROBES FOR RAPID CHARACTERIZATION OF HUMAN CHROMOSOMES IN HYBRID CELL LINES" PROCEEDINGS OF THE NATIONAL ACADEMY OF SCIENCES OF USA, Bd. 87, Nr. 17, 1. September 1990 (1990-09-01), Seiten 6634-6638, XP000310538 ISSN: 0027-8424

## Beschreibung

Die Erfindung betrifft neue Oligonukleotidsequenzen, ein Verfahren zur Durchführung einer Inter-LINE-PCR unter Verwendung von Oligonukleotiden mit diesen Sequenzen und die Verwendung der Oligonukleotide als Primer für eine Inter-LINE-PCR, insbesondere zur Diskriminierung von DNA der Mitglieder der Prokarya, Archaea oder Eukarya oder genetisch veränderter Organismen hiervon.

Durch die Polymerase-Kettenreaktion (Polymerase chain reaction, PCR) kann ein definiertes DNA-Fragment unter Verwendung von flankierenden komplementären Oligonukleotiden, sog. Primer, aus einem komplexen DNA-Gemisch amplifiziert werden. Voraussetzung für die Konstruktion solcher Primer ist die Kenntnis der zu amplifizierenden DNA-Sequenz. Werden hingegen bei der PCR Primer von kurzer zufälliger Sequenz eingesetzt, kann aus genomischer DNA eine ganze Reihe von unbekannten DNA-Abschnitten amplifiziert werden. Die verschiedenen amplifizierten DNA-Fragmente können dann gelelektrophoretisch aufgetrennt werden und sind der weiteren Analyse zugänglich. Diese in jüngster Zeit zunehmend angewandte Technik wird als 'arbitrary PCR' (AP-PCR) bezeichnet (Übersicht Tab.1). Der Informationsgehalt einer solchen PCR-Anwendung kann wesentlich erhöht werden, wenn statt zufällig gewählter Primersequenzen solche verwendet werden, die von repetitiven, weit im Genom verbreiteten DNA-Elementen abgeleitet sind (Nelson et al., 1989; Ledbetter & Nelson, 1991; Munroe et al., 1994; Zietkiewicz et al., 1994).

LINE's ("long interspersed elements") bilden eine Klasse solcher verstreut im Genom liegenden, repetitiven DNA-Elemente mit ca. 100.000 Kopien pro Säugergenom. Eine vollständige LINE-Sequenz besteht aus ca. 6.300 Basenpaaren; die meisten Elemente, die im Genom vorkommen, sind jedoch verstümmelt. Insgesamt repräsentieren sie etwa 10% der DNA des Säugergenoms (Richard et al., 1994). Die komplette LINE-Sequenz beeinhaltet zwei offene Leserahmen (ORF), wobei ORF2 für Reverse Transkriptase, eine retrovirale Polymerase, kodiert (Hattori et al., 1986; Mathias et al., 1991). Die Existenz der Reversen Transkriptase sowie weitere strukturelle und funktionelle Ähnlichkeiten mit Retrotransposons deuten auf einen viralen Ursprung dieser Elemente hin. Vergleichbar den Retroviren sind auch komplette LINEs zur duplizierenden Transposition befähigt (Katzir et al., 1985; Miki et al., 1992). LINE-verwandte Sequenzen sind im Tier- und Pflanzenreich weit verbreitet, und deren ausgeprägte Sequenzhomologie zwischen verschiedenen Spezies läßt auf ein hohes evolutionäres Alter dieser Elemente schließen. Ihre Mobilität ist streng reguliert, was während der Evolution zur speziesspezifischen Verteilung im Genom geführt hat. Andererseits kann durch DNA-Schädigung oder Zellstreß die vermehrte Aktivierung der Transposition initiiert werden, und dies führt zu einer veränderten Anzahl und Verteilung von LINEs im Genom (Servomaa & Rytömaa, 1990).

Es ist eine Aufgabe der vorliegenden Erfindung, Oligonukleotide bereitzustellen, die zur Analyse und Charakterisierung der Genome verschiedener Spezies sowie zur Konstruktion von Markern für die Tumordiagnostik geeignen.

Diese Aufgabe wird erfindungsgemäß durch die Bereitstellung von Oligonukleotiden gelöst, die aus einer oder mehrerer der nachfolgenden Oligonukleotide ausgewählt werden:

Die Aufgabe der Erfindung wird auch dadurch gelöst, daß diese Oligonukleotide am 3'-Ende und/oder am 5'-Ende um eine oder mehrere Nukleotide verkürzt sind. Die verkürzten Oligonukleotide sollen jedoch mindestens 15 Nukleotide umfassen. Die Verkürzung wird bevorzugt am 5'-Ende durchgeführt, wobei jedoch auch Verkürzungen am 3'-Ende möglich sind. Die Verkürzung kann entweder am 3'-Ende oder am 5'-Ende oder an beiden Enden erfolgen.

In einer weiteren Ausführungsform der Erfindung ist das 5'-Ende der Oligonukleotide modifiziert. Die Modifikationen der erfindungsgemäßen Oligonukleotide können aus beliebigen Modifikationen ausgewählt werden, die aus dem Stand der Technik bekannt sind. Bevorzugte Modifikationen umfassen die Verknüpfung der Oligonukleotide am 5'-Ende mit Aminosäuren, mit Digoxigenin, mit Biotin und mit weiteren, zusätzlichen Nukleotiden. Diese weiteren zusätzlichen Nukleotide bilden bevorzugt eine oder mehrere Erkennungsstellen für Restriktionsendonukleasen.

Die Erfindung umfaßt auch solche Oligonukleotide, die zu den obengenannten sieben Oligonukleotidsequenzen eine Homologie aufweisen, die bei ≥ 95 % liegt, in einer besonders bevorzugten Ausführungsform bei ≥ 97 %.

Die Oligonukleotide der vorliegenden Erfindung können, wie oben näher ausgeführt, in verschiedener Weise abgeändert werden. Die Abänderungen sollen jedoch bevorzugt so durchgeführt werden, daß der Anteil der in den Oligonukleotiden verbleibenden Guanin- und Cytidinnukleotide 50 % ± 5 % beträgt.

Die Erfindung umfaßt auch beliebige, aus dem Stand der Technik bekannte Vektorsysteme, die die erfindungsgemäßen Oligonukleotide enthalten.

Die erfindungsgemäß bereitgestellten Oligonukleotide werden in einer Polymerase-Kettenreaktion eingesetzt. Eine derartige Polymerase-Kettenreaktion wird als Inter-LINE-Polymerase-Kettenreaktion, abgekürzt IL-PCR, bezeichnet. Die Durchführung einer IL-PCR umfaßt zumindest die nachfolgenden Verfahrensschritte:
a) Vermischen der zu analysierenden DNA mit einem oder mehreren Oligonukleotiden mit einer Sequenz nach einem oder mehreren der Ansprüche 1 bis 7 in einem zur Durchführung einer PCR-Reaktion geeigneten Reaktionspuffer und wahlweise weiteren Zusätzen;
b) Durchführung von mehrere De- und Renaturierungen umfassenden Amplifikationsschritten zur Gewinnung amplifizierter DNA-Produkte; und
c) Auftrennung und Analyse der Amplifikationsprodukte.

Die Durchführung einer Polymerase-Kettenreaktion ist dem Fachmann aus einer Vielzahl von Veröffentlichungen bekannt. Nur beispielsweise sei auf die in der beiliegenden Literaturliste genannten Publikationen hingewiesen. Abänderungen an der Poly-merase-Kettenreaktion sind zulässig, um die Verfahrensbedingungen für eine spezifische Anwendung zu optimieren. Derartige Optimierungen sind für den Fachmann ohne weitere erfinderische Schritte ausführbar und werden von der vorliegenden Erfindung umfaßt,

Die mit den erfindungsgemäß bereitgestellten Oligonukleotiden durchgeführte IL-PCR umfaßt bevorzugt einen ersten Denaturierungsschritt, einen odere mehrere De- und Renaturierungen mit niedriger Stringenz und mehrere De- und Renaturierungen mit höherer Stringenz und einen abschließenden Kettenverlängerungsschritt als Amplifikationsschritte.

Bevorzugt werden 3 - 7 De- und Renaturierungsschritte mit niedriger Stringenz und 20 - 30 De- und Renaturierungsschritte mit höherer Stringenz durchgeführt.

Die Reaktionspuffer zur Durchführung der PCR-Reaktion sind dem Fachmann bekannt und können in üblicher Weise an die Erfordernisse des durchzuführenden Experiments angepaßt werden. Bevorzugt enthalten die Reaktionspuffer ein Mittel zur Viskositätserhöhung, insbesondere bevorzugt Gelatine.

Die Analyse der PCR-Produkte erfolgt durch übliche, dem Fachmann bekannte Trennverfahren, beispielsweise durch ein Agarosegel oder Polyacrylamidgel.

Die erfindungsgemäß bereitgestellten Oligonukleotide werden als Primer für eine Inter-LINE-Polymerase-Kettenreaktion eingesetzt. Durch die Verwendung der erfindungsgemäßen Oligonukleotide in einer PCR-Reaktion ist es möglich, DNA der Prokarya, Archaea oder Eukarya oder genetisch veränderte Organismen hiervon zu charakterisieren bzw. zu diskriminieren. Beispielsweise ist die Diskriminierung bzw. Charakterisierung einzelner Taxa und Stämme der Prokarya, Archaea oder Eukarya möglich. Beispielsweise sind Hefen und Bakterien durch das erfindungsgemäße IL-PCR-Verfahren unterscheidbar.

In einer weiter bevorzugten Ausführungsform werden die erfindungsgemäß bereitgestellten Oligonukleotide eingesetzt, um Tumorzellen von normalen Zellen zu unterscheiden. Durch das erfindungsgemäße Verfahren können Transformations- und/oder Tumormarker konstruiert und identifiziert werden. Die erfindungsgemäß bereitgestellten Oligonukleotide werden somit bevorzugt in der Tumordiagnostik eingesetzt. Weitere Einsatzgebiete und Anwendungen sind in den nachfolgenden Ausführungsbeispielen genannt.

Die für eine Inter-LINE-PCR verwendbaren Oligonukleotide ergeben sich dadurch, daß zumindest die nachfolgenden Schritte durchgeführt werden:
1. Auswählen beliebiger LINE-Sequenzen;
2. Alignment dieser LINE-Sequenzen;
3. Bildung von Konsensus-Sequenzen aus konservierten Abschnitten dieser LINE-Sequenzen durch Auswählen solcher Bereiche, die die nachfolgenden Kriterien erfüllen:
   a) mindestens 20 Basenpaare mit Homologiewerten ≥ 80%, bevorzugt ≥ 90%, zu LINE-Sequenzen;
   b) einem CG:AT-Verhältnis von ≥ 1;
   c) GC-reiche 5'- und 3'-Enden; und
   d) Auswählen solcher Nukleotide, die in vertikaler Ebene des Alignments am häufigsten auftreten.

Die LINE-Sequenzen sind bevorzugt dadurch definiert, daß sie zwei offene Leserahmen (ORF) aufweisen, die für eine reverse Transkriptase und eine retrovirale Polymerase oder Teile hiervon codieren. LINE-Sequenzen mit anderen Merkmalen sind ebenfalls einsetzbar.

Die erfindungsgemäß entwickelte Technik, die sogenannte Inter-LINE-PCR (IL-PCR) kann somit in überraschend hervorragender Weise zur Analyse und Charakterisierung der Genome verschiedener Spezies benutzt werden.

Die verwendeten Primer wurden von Säuger-DNA-Sequenzen abgeleitet und wurden ursprünglich zur Analyse von Säugergenomen verwendet, da mit jedem Primer Spezies-spezifische, reproduzierbare Muster der PCR-amplifizierten Fragmente für verschiedene Zellinien, z.B. von Hamster, Maus, Mensch etc., erstellt werden konnten. Unter bestimmten Bedingungen sind dieselben Primer, auch für die Charakterisierung und Identifizierung von niederen Eukaryonten (wie z.B. Hefen), Archebakterien und Prokaryonten geeignet, obwohl zumindest bei den Archebakterien und Prokaryonten keine LINE-verwandten Sequenzen bekannt sind. Nach elektrophoretischer Auftrennung der IL-PCR Produkte entstehen spezifische Muster von Banden, durch die auch nahe verwandte Gattungen, Arten oder Stämme diskriminiert werden können.

Nach einer vorausgehenden Sequenzanalyse und Vergleich der Sequenzen können gezielt PCR-Primer für Mikroorganismen synthetisiert werden, die spezifisch definierte Taxa erfassen. Das Anwendungsgebiet dieser Methode ist so groß, daß durch Einsatz konservativer Primer Mitglieder der drei Domänen Bacteria, Archaea und Eukarya erfaßt und andererseits selbst nah verwandte Arten diskriminiert werden können. Mit Hilfe von Hybridisierungstechniken und/oder spezifischer PCR ist ein routinemäßiger Einsatz zur Identifikation verschiedenster Organismen und Zellkulturen möglich.

Im Gegensatz zu bisher etablierten Techniken, die in Tabelle 1 aufgelistet sind, können Organismen auf verschiedenen taxonomischen Ebenen, beispielsweise Gattung, Art und Stamm, identifiziert und charakterisiert werden. Alle anderen Methoden sind auf ein bis zwei dieser Niveaus limitiert.

Die Erfindung wird nunmehr anhand der beiliegenden Abbildungen näher erläutert. Die Abbildungen zeigen:
- Abbildung 1: Vergleich verschiedener PCR-Anwendungen;
- Abbildung 2: Position der LINE-homologen Klone zur Ratten-LINE-Sequenz;
- Abbildung 3: Alignment einiger LINE-homologen Klone in Bereich der Rat LINE Sequenz Pos. 3220-3346
- Abbildung 4: Alignment-Ausschnitt für Konstruktion der Primer GF und GR
- Abbildung 5: Alignment einiger LINE-homologen Klone im Bereich der Rat-LINE-Sequenz Pos. 4611-4745
- Abbildung 6: Alignment-Ausschnitt zur Konstruktion der Primer RF und RR

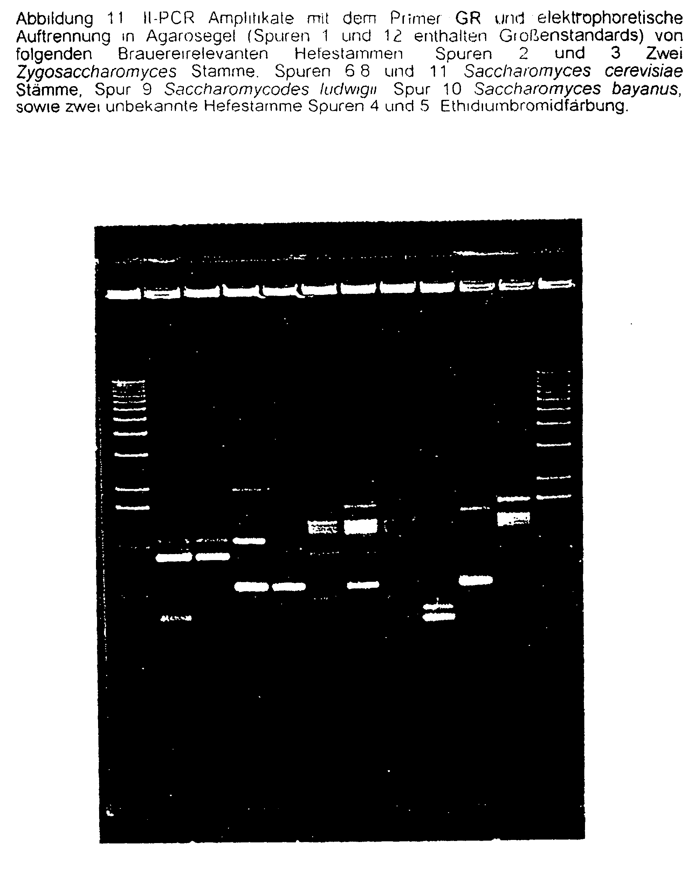

- Abbildung 7 - Abbildung 16:: Verschiedene Anwendungen der IL-PCR

### ALLGEMEINE BESCHREIBUNG DER ERFINDUNG

Im Rahmen eines Forschungsprojekts zur Aufklärung der molekularen Mechanismen der Strahlenkarzinogenese sollte die Bedeutung von genetischen Veränderungen in neoplastisch transformierten Embryofibroblasten des Syrischen Hamsters (SHE-Zellen) untersucht werden. Hierzu wurden Bibliotheken von genomischer DNA von normalen (nicht transformierten) und von transformierten Zellen konstruiert und die DNA-Bibliotheken mittels differentieller Hybridisierung verglichen. Klone, die in einer der Bibliotheken über- bzw. unterrepräsentiert waren, wurden isoliert und sequenziert. Dabei konnte eine Reihe von Genen isoliert werden, die bei der Karzinogenese von Bedeutung sind. Bei dieser Analyse waren auffällig viele Klone mit LINE-homologen Sequenzen isoliert worden.

Aus der Konsensus-Sequenz dieser LINE-Klone wurden mehrere universelle PCR-Primer abgeleitet, die einzeln oder in Kombination in die Inter-LINE-PCR eingesetzt wurden. Für die Amplifikation wurde zunächst DNA aus SHE-Zellen, später auch DNA verschiedener anderer Säuger-Spezies, verwendet. Die elektrophoretische Auftrennung der Amplifikate in Agarose- bzw. Polyacrylamid-Gelen ergab charakteristische Bandenmuster der Länge zwischen 100 und 5000 bp, die nachweislich organismusspezifisch sind.

Erfindungsgemäß konnte gezeigt werden, daß bei geeigneten PCR-Bedingungen selbst bei niederen Eukaryonten (Hefen) und bei Prokaryonten wie Bakterien (E.coli, verschiedenen Bodenbakterien, sowie den rasch evolvierenden Planctomyceten) und bei Archaeas spezifische Bandenmuster entstehen, obwohl diese Organismen nach bisheriger Kenntnis keine LINEs besitzen. Insbesondere konnte nachgewiesen werden, daß diese Technik für die Differenzierung verschiedener Hefearten geeignet ist, wie sie zum Beispiel in der Brauereitechnologie, in der Weinwirtschaft oder in der Bäckerei eingesetzt werden. Ebenso ist es möglich schädliche Prokaryonten, die in der Brauereitechnologie auftreten, als Spurenverunreinigungen zu erkennen und die verursachenden Schädlinge zu identifizieren.

Zusätzlich konnte in Erweiterung der ursprünglichen Arbeiten gezeigt werden, daß sich bei genauer Analyse auch die Bandenmuster der PCR-Produkte von normalen und tumorogenen Säugertierzellinien im Detail voneinander unterscheiden lassen. Veränderungen der Bandenmuster (fehlende, zusätzliche oder in ihrer Länge variierenden Banden) können als Indikatoren bei der Diagnose der frühen Ereignise in der Tumorentwicklung dienen, wie das Beispiel der strahleninduzierten Transformanten von embryonalen Fibroblasten des Syrischen Hamsters belegt.

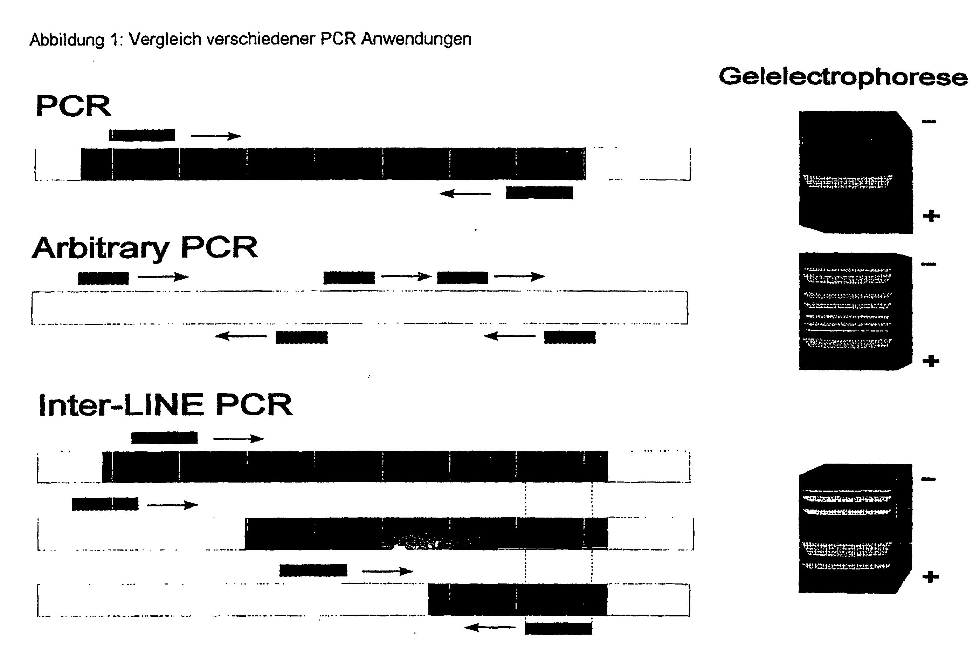

In der Abbildung 1 wird die Inter-LINE-PCR schematisch im Vergleich zu klassischen PCR-Anwendungen gezeigt. Die graugefüllten Balken stellen Bereiche von bekannter Basensequenz dar. Mit Pfeilen wurde die Syntheserichtung der Polymerase (ausgehend von dem jeweiligen Primer) eingezeichnet. Auf der rechen Seite der Zeichnung wurden schematisch die erwarteten PCR Produkte auf Agarose-Gelen aufgetrennt. Besonders hervorzuheben sind die Qualitätsunterschiede der Amplifikationsprodukte in Vergleich zwischen der AP-PCR Anwendung und der Inter-LINE-PCR. Die Inter-LINE-PCR Produkte bestehen zum Teil aus bekannten Sequenzbereichen. Aufgrund dieser Tatsache werden diese Produkte für weitergehende molekularbiologische Analysen zugänglich gemacht.

### DETAILIERTE BESCHREIBUNG DER ERFINDUNG

Die Erfindung wird im folgenden anhand einiger Anwendungsbeispiele im Detail beschrieben, wobei die Anwendung dieser Technik nicht auf diese Beispiele limitiert ist.

### A. Grundlegende Vorarbeiten, die für alle beschriebenen Anwendungen identisch bzw. Voraussetzung sind.

### a) Konstruktion von genomischen DNA-Bibliotheken

Mit Restriktionsenzym EcoRI verdaute genomische Gesamt-DNA von embryonalen Fibroblasten des Syrischen Hamsters (SHE-Zellen) wurde im Lambda gt11 Vektor kloniert. Die Vermehrung der rekombinanten Phagen erfolgte im Y1088-Derivatstamm von E. coli K12 (Pharmacia, Freiburg). Klonierung und Verpackung der Phagen wurde nach standardisierten molekularbiologischen Methoden (Sambrook et al., 1989) durchgeführt.

### b) Differentielle Hybridisierung der rekombinanten Klone

Jeweils annähernd 1000 rekombinante Phagenklone wurden mit "Overlayer"-Technik (Sambrook et al., 1989) ausplattiert. Es wurden pro Platte zwei identische "Plaque-Blots" hergestellt. Diese wurden dann nacheinander mit homologen (aus normalen SHE-Zellen) und heterologen (SHE-Tumorzellen) genomischen DNA-Proben hybridisiert. Rekombinante Klone, die auffällige Unterschiede im Hybridisierungs-verhalten zeigten, wurden isoliert. Parallel zur oben beschriebenen Technik wurden auch cDNA-Proben von Gesamt-mRNA der beiden zu vergleichenden Zellinien als Hybridisierungsproben eingesetzt. Hierbei wurden alle Klone isoliert, die Hybridisierungssignale zeigten.

### c) Charakterisierung der rekombinanten Klone

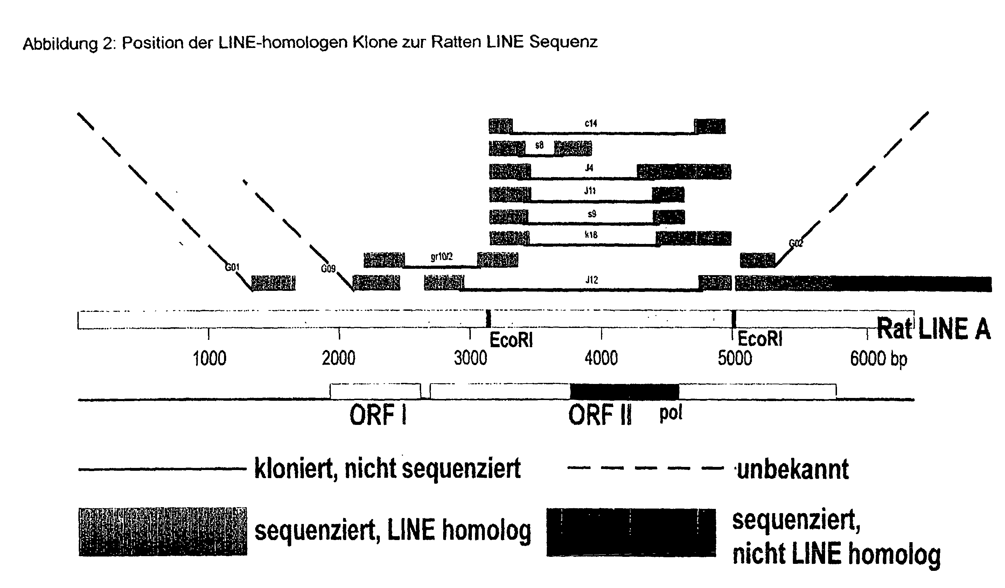

Zwanzig der auf diese Weise isolierten Klone wurden mit T7 Polymerase und Vektor gtll spezifischen Primern sequenziert (Pharmacia). Die DNA-Sequenzen wurden dann mit Hilfe des HUSAR-Computerprogramms (DKFZ, Heidelberg) analysiert und mit Datenbanken (Genius-Genbank, Heidelberg) verglichen. Zwölf der zwanzig Klone zeigten eine signifikante Übereinstimmung mit bereits bekannten Nagetier-LINE-Sequenzen. In der Abbildung 2 werden diese Klone mit ihrer relativen Position zu der Ratten-LINE-DNA-Sequenz (RNLINED,Genebank Heidelberg) gezeigt.

### d) Konstruktion der LINE-spezifischen PCR-Primer

Wie aus der Abbildung 2 ersichtlich, ist die Mehrzahl der isolierten Klone zu dem Bereich der Ratten-LINE- Sequenz homolog, der zwischen den beiden EcoRI Schnittstellen liegt und der dem am höchsten konservierten Bereich der LINEs entspricht. Dieser Bereich beinhaltet u.a. den für die Reverse Transkriptase homologen Sequenzbereich. Sequenzen solcher Klone werden zum Teil in den Abbildungen dargestellt. Alignement dieser Sequenzen, das mit Hilfe des HUSAR-Programms erstellt wurde, weist Bereiche von unterschiedlich ausgeprägten Ähnlichkeiten in der Basenabfolge auf. Solche Bereiche, die höchste Homologiewerte zeigten, wurden auf ihre Eignung zur Primerkonstruktion hin überprüft. Folgende Kriterien wurden bei dieser Auswahl beachtet:
1. Mindestens 20 Basenpaare mit hohen Ähnlichkeitswerten,
2. CG:AT-Verhältnis soll mindestens 1 bzw. mehr als 1 betragen,
3. Die 5'- und 3'-Enden dieser Bereiche sollen GC-reich sein, um die relative Bindungsenergie der konzipierten Primer an diesen Stellen zu erhöhen

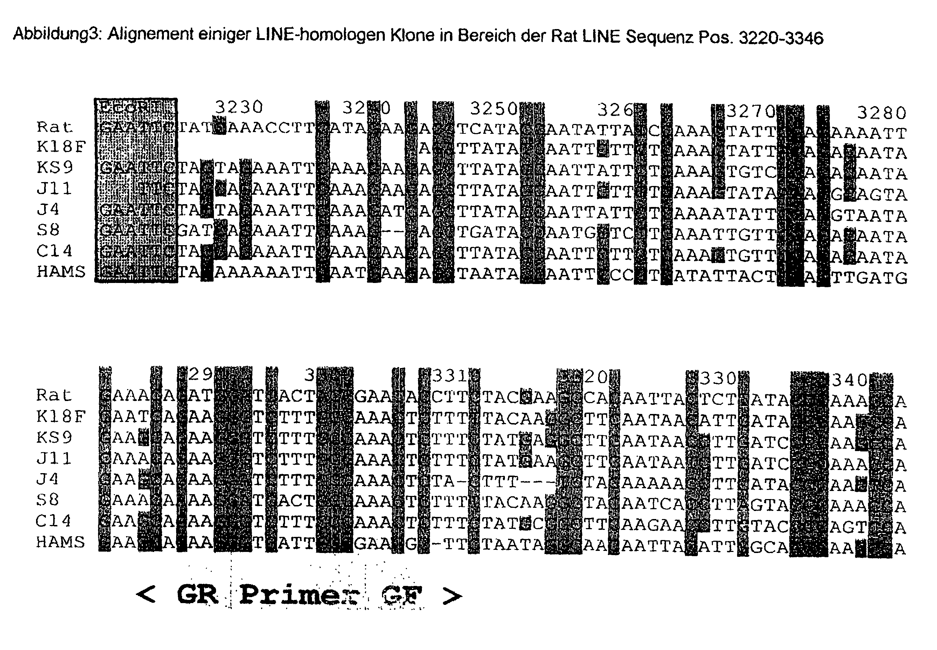

Die Abbildung 3 zeigt das Alignement der Sequenzen, die in dem Bereich von der EcoRI-Schnittstelle (Rat LINE Position 3220-3226) bis zu Rat-LINE-Position 3346 ermittelt wurden. Zur Verdeutlichung der Auswahlkriterien für die Primerkonstruktion wurden die GC-reichen, konservierten Positionen grau unterlegt.

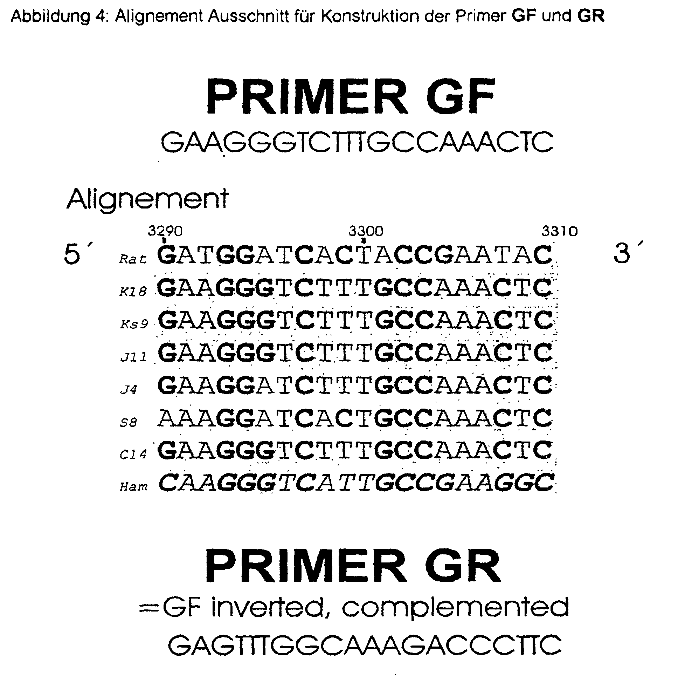

In der Abbildung 4 ist der Abschnitt des Alignements dargestellt, der am besten den von uns aufgestellten Kriterien entsprach und der folglich zur Primerkonstruktion verwendet wurde. Die homologen, d.h. mit identischen Basen besetzten Positionen wurden grau unterlegt. Die Konsensussequenz und die entsprechende invertierte Komple-mentärsequenz wurden dann letztendlich für die Synthese der Primer-Oligonukleotide verwendet.

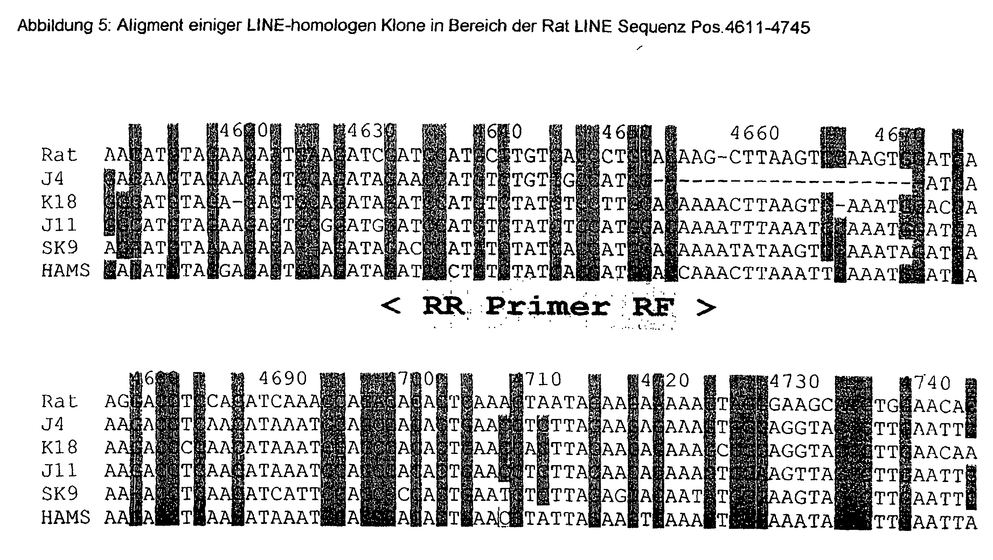

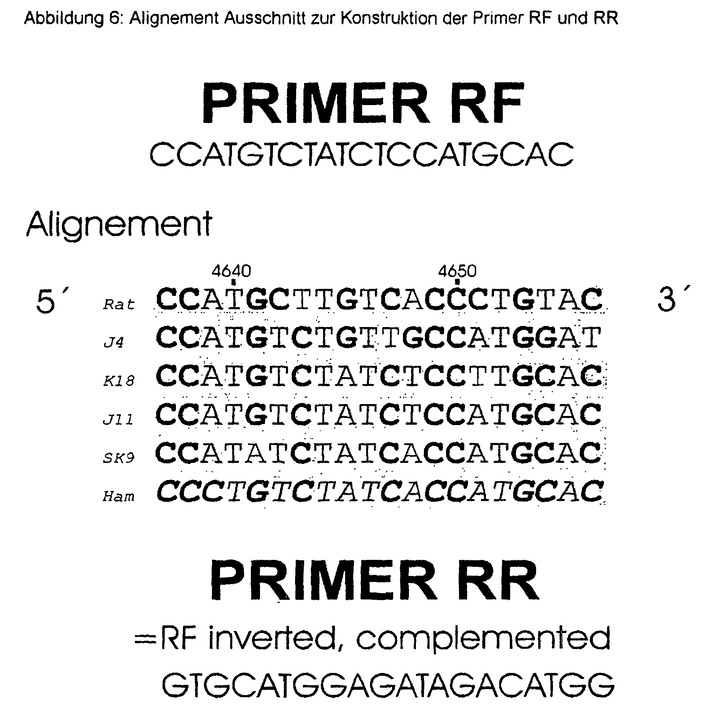

Die Abbildungen 5 und 6 stellen analog zur Konstruktion der Primer **GF** und **GR** die Konstruktion der Primer **RF** und **RR** dar, die aus dem Alignement im Bereich 3290-3310 (Rat LINE Positionen) konstruiert wurden.

Die folgende Liste enthält solche Primer, die für die IL-PCR einzeln oder in Kombination verwendet wurden. Zusätzlich zu den bereits beschriebenen Konsensus-Oligonukleotiden wurden in diese Liste auch solche Primer aufgenommen, die direkt aus den Sequenzen einiger LINE-Klone konstruiert wurden und ebenfalls unsere Kriterien erfüllen. Verkürzte Primer (zB. **GRkurz**) können insbesondere bei der Genomanalyse von Prokaryonten eingesetzt werden.

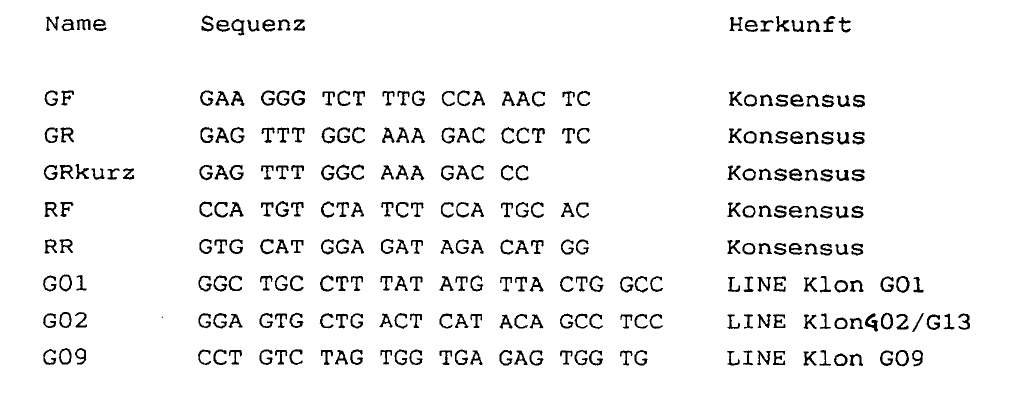

### e) Inter-LINE-PCR

### Reaktionsbedinqunqen für die PCR-Amplifikation

Die Reagenzien wurden den Herstellerangaben (GIBCO BRL, Eggenstein) entsprechend im Gesamtvolumen von 50 µl eingesetzt. Dem 10fach PCR-Puffer wurde 0,005% Gelatine zugesetzt. 200 pMol Oligonukleotid und 100 ng genomische DNA wurden pro 50 µl-Reaktionsansatz verwendet. Die PCR-Reaktion wurde im Hybaid-Heat Block (MWG, Ebersberg) durchgeführt.

### Amplifikationsparameter

| | | |
|---|---|---|
| Erster Denaturierungsschritt | 8 min | 93°C |
| | | |
| 5 Zyklen mit niedrigerer Stringenz | 1 min 30s | 37°C |
| | 2 min | 72°C |
| | 1 min 30s | 93°C |
| 25 Zyklen mit höherer Stringenz | 1 min | 52°C |
| | 1 min 30s | 72°C |
| | 1 min | 93°C |
| | | |
| Finale Kettenverlängerung | 10 min | 72°C |

Zur Kontrolle und Analyse der Amplifikationsprodukte wurden diese elektro-phoretisch in 1%igen Agarosegelen aufgetrennt und durch Ethidiumbromidfärbung sichtbar gemacht.

### Polyacrylamid-Gelelektrophorese

6%-ige, nicht denaturierende PAA-Gele (9 ml Acrylamidstammlsg.: 300 g Acrylamid und 5g N,N'-Methylen-bisacrylamid/l H₂O; 0,9 ml ME-Puffer: 1 M MOPS, 10 mM EDTA,
pH 8,0; und 11,25 ml 40 % Glycerol mit H₂O auf 45 ml Gesamtvol. auffüllen; + 75 µl TEMED; + 50 µl APS) wurden auf einer hydrophilen Trägerfolie den Herstellerangaben (DIAGEN, Hilden) entsprechend gegossen. Die Elektrophorese erfolgte in einer Temperatur-Gradienten-Gelelektrophorese-Apparatur der Firma Diagen, Hilden (TGGE) mit lx MOPS-Laufpuffer bei konstant 25°C, 3-4 h bei 200 V. Die Gele wurden nach dem Protokoll der Fa. Diagen mit Silbernitrat gefärbt und fotografisch dokumentiert.

### B. Einige Beispiele für die Anwendung der Erfindung

### Beispiel 1:

### Identifizierung von neoplastisch transformierten Zellinien und Ableitung von Transformations- bzw.Tumormarker

Mit Hilfe der Inter-LINE-PCR können neoplastisch transformierte Zellinien des Syrischen Hamsters und daraus in der Nacktmaus etablierte Tumorzellinien identifiziert und von normalen SHE-Zellen unterschieden werden.

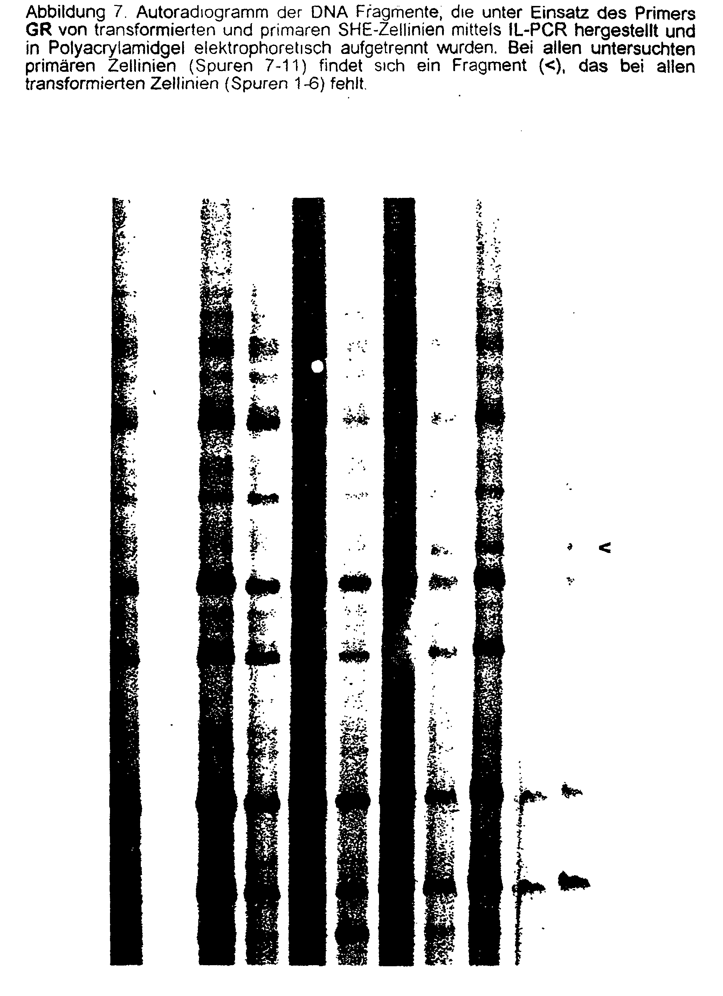

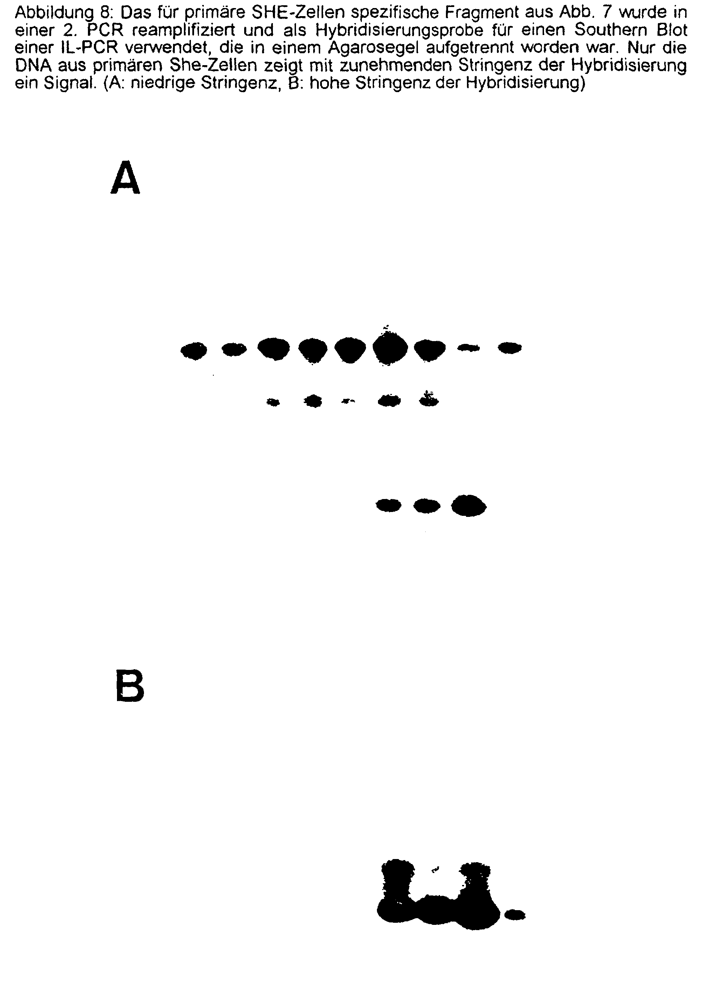

Primäre Embryofibroblasten des Syrischen Hamsters wurden durch Exposition mit ionisierender Strahlung neoplastisch transformiert. Injiziert man diese transformierten Zellinien in die athymische Nacktmaus, so entwickeln sich Tumoren; nach deren Isolation können daraus Tumorzellinien etabliert werden. Die genomische DNA aus den verschiedenen Zellinien wurde mittels Standardmethoden isoliert. Unter Einsatz der beschriebenen Primer wurden spezifische DNA-Fragmente durch die Inter-LINE-PCR amplifiziert. Die Amplifikate wurden radioaktiv markiert und elektro-phoretisch in Polyacrylamidgel aufgetrennt. Wie in Abb.7 dargestellt können transformierte Zellinien von normalen SHE-Zellen unterschieden werden, in diesem Fall anhand einer fehlenden bzw. zusätzlichen Bande. Diese Bande wurde dann isoliert, reamplifizert, radioaktiv markiert und als Sonde für einen Southern-Blot der IL-PCR eingesetzt (Abb.8). Grundsätzlich gilt, daß die Bandenmuster der IL-PCR-Amplifikate der Säuger-Genome sehr komplex und auf Agarosegelen nicht differenzierbar sind; in der Southern-Analyse (als geeignete Sonden können IL-PCR-Produkte, LINE-Klone oder auch Microsatelliten probes eingesetzt werden) können hingegen tumorspezifische bzw. transformations-spezifische Banden erkannt werden. Nach weiterer Charakterisierung können diese in Zukunft als Transformations- bzw. Tumormarker für den diagnostischen Einsatz verwendet werden.

### Beispiel 2:

### Unterscheidung verschiedener Säugerspezies

Mittels der Inter-LINE-PCR können einzelne Säugerspezies anhand des Bandenmusters der PCR-Amplifikate unterschieden werden. Hierzu kann sowohl genomische DNA aus Gewebeproben der Tiere als auch DNA aus etablierten Zellinien benutzt werden.

Die genomische DNA wurde mittels Standardmethoden aus den Zellkulturen bzw. den Gewebeproben isoliert. Für die PCR-Amplifikation wurden die Primer **GF, GR, RF** und **RR** eingesetzt; die PCR-Bedingungen waren entsprechend wie unter e) beschrieben.

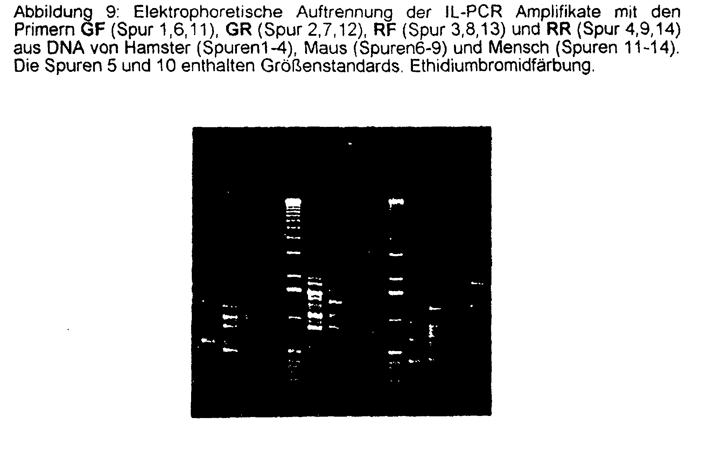

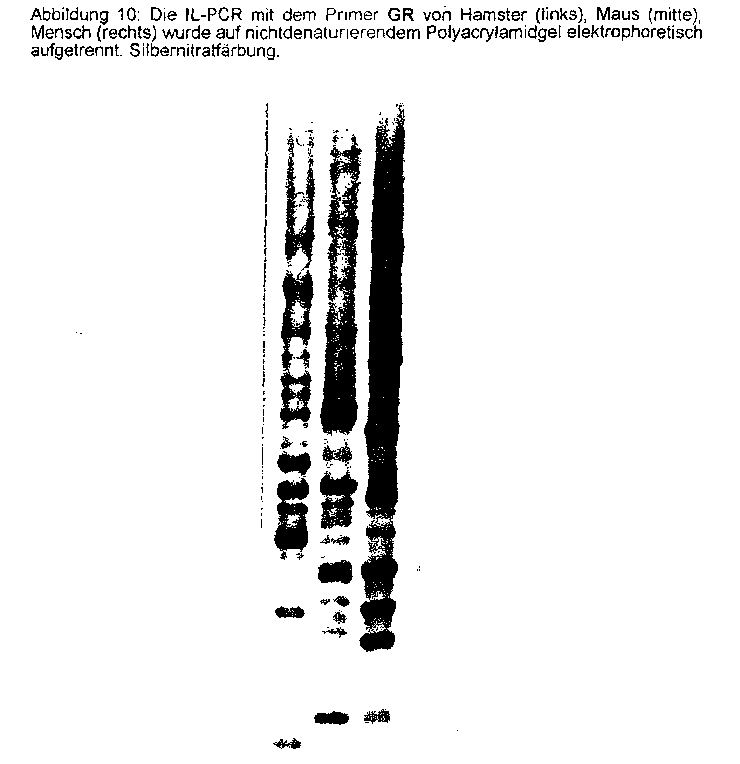

Die PCR-Amplifikate wurden sowohl in Agarose- als auch in Polyacrylamidgelen aufgetrennt. Abb.9 und Abb.10 zeigen die klar unterscheidbaren Bandenmuster von Hamster, Maus und Mensch als Beispiele.

### Beispiel 3:

### Identifizierung und Diskriminierung von verschiedenen Hefestämmen

Die Identifizierung und Unterscheidung einzelner Hefestämme mittels der Erfindung der IL-PCR wird am Beispiel der in der Brautechnologie relevanten Nutz- und Schadhefen demonstriert.

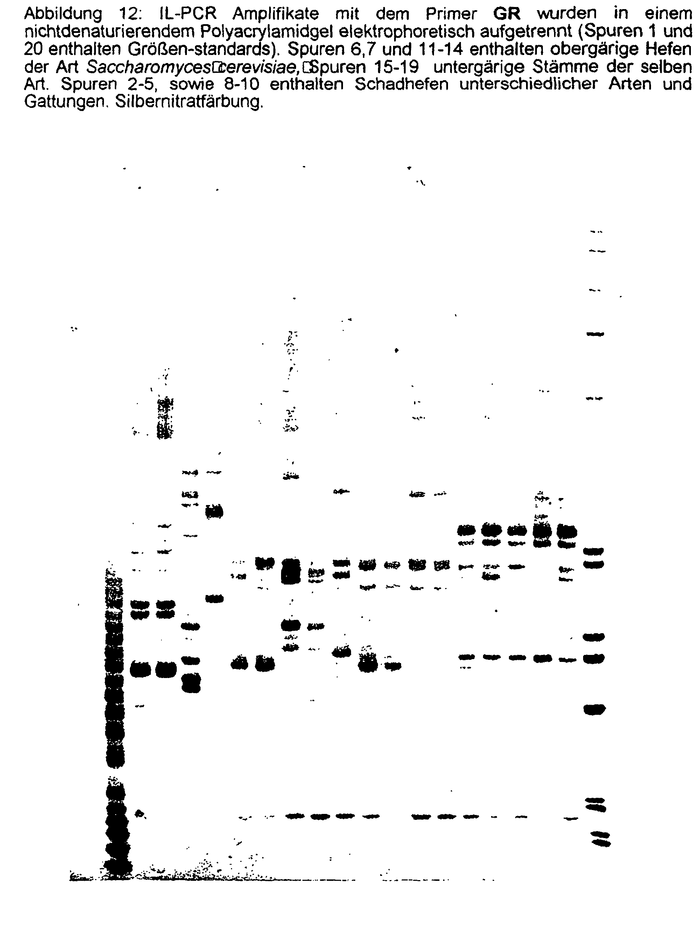

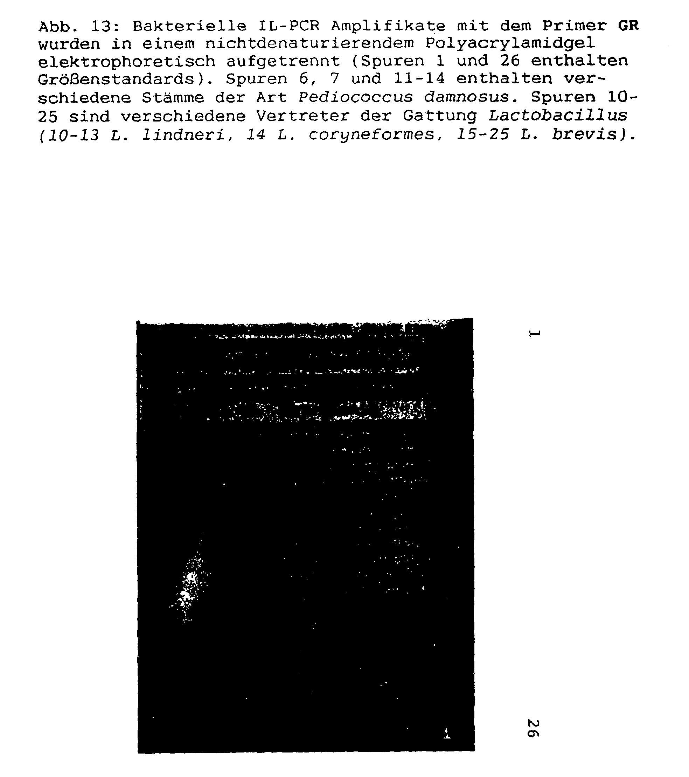

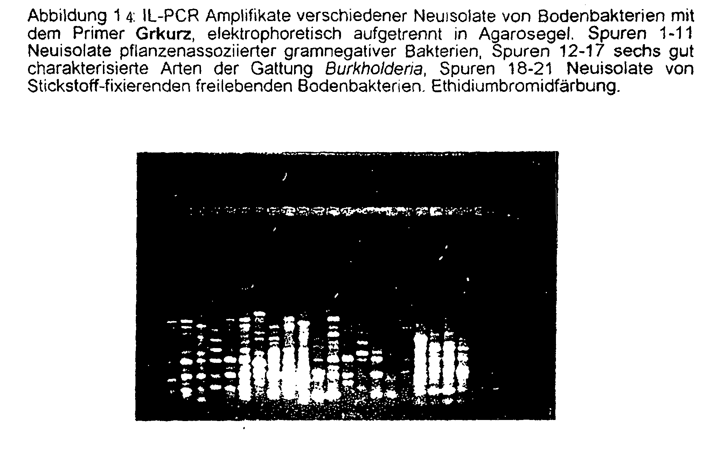

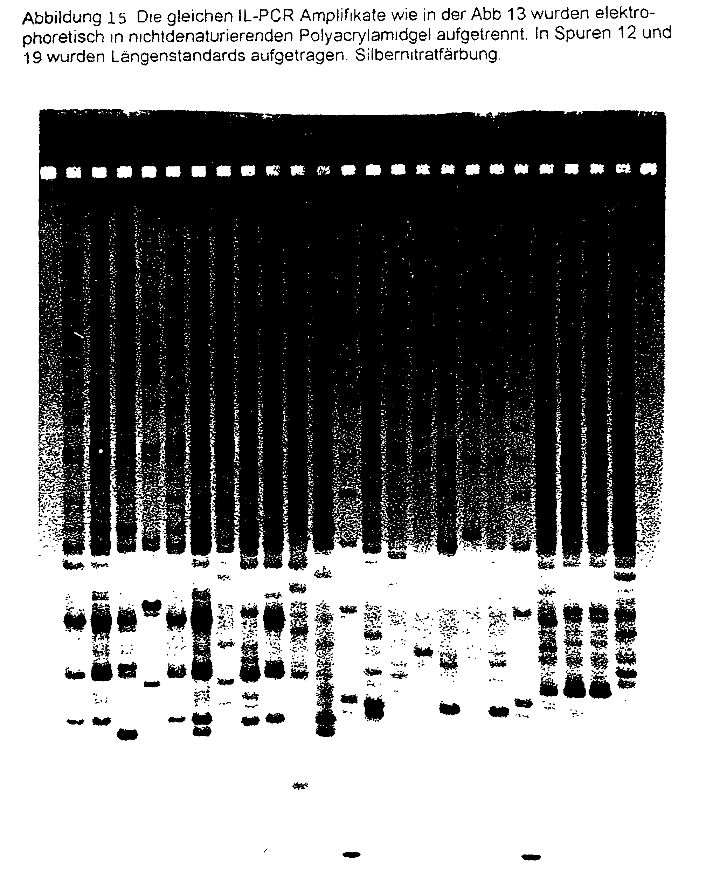

100 ng der nach Standardprotokollen isolierten genomischen Hefe-DNA wurden in der Inter-LINE-PCR amplifiziert (siehe e). Die PCR-Amplifikate derselben IL-PCR-Reaktionen wurden sowohl in Agarosegelen (Abb.ll) als auch in Polyacrylamidgelen (Abb. 12, Hefen; Abb. 13, Bakterien) aufgetrennt. Anhand der unterschiedlichen Bandenmuster können die untersuchten Hefen und Bakterien auf Gattungs- und Artniveau, teilweise auch auf Stammebene, differenziert werden. So können beispielsweise Schadhefen im Bierbrauprozess klar von den Nutzhefen unterschieden werden.

### Beispiel 4:

### Taxonomische Analyse von Prokaryonten

Am Beispiel der Identifizierung und taxonomischen Zuordnung von Neuisolaten von Bodenbakterien wird das hohe Anwendungspotential der Erfindung der IL-PCR demonstriert.

Die genomische DNA aus neuisolierten Bodenbakterien unbekannter Art und Gattung wurde nach Standardmethoden (Sambrook et al.,1989) gewonnen. Für die IL-PCR in dieser Anwendung wurde ein 3'-verkürztes Oligonukleotid, **GRkurz**, als Primer eingesetzt. Die PCR-Bedingungen waren entsprechend wie unter e) beschrieben. Die Amplifikate wurden für die Analyse sowohl in 1%igen Agarosegelen (Abb.14: Eine ganze Reihe von pflanzenassoziierten Neuisolaten aus Reiskulturen wurde mit bereits charakterisierten Typusstämmen verglichen) und in Polyacrylamidgelen (Abb.15: gleiche IL-PCR wie in Abb. 14) elektrophoretisch aufgetrennt. Die Neuisolate können anhand des PCR-Musters in a) taxonomische Gruppen eingeordnet werden, b) Parallelisolate detektiert und c) falls Typus bereits bekannt, auch identifiziert werden.

### Beispiel 5:

### Nachweis von Mycoplasmenkontaminationen in Säugetierzellkulturen

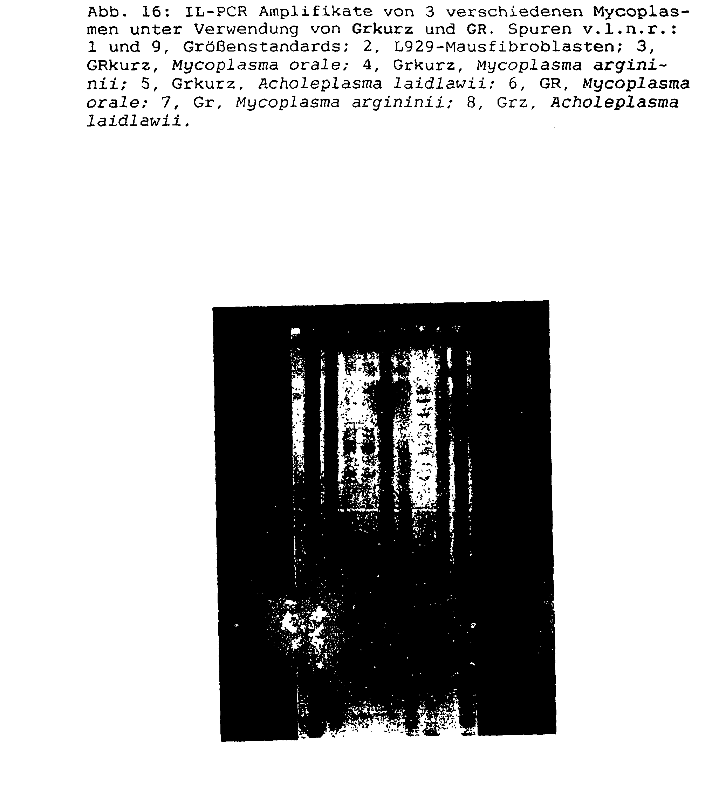

Aus dem Nährmedium von mit Mycoplasmen infizierten L929 Mausfibroblasten wurden die Mycoplasmen isoliert und nach Standardmethoden (Sambrook et al., 1989) DNA isoliert. Mittels der Oligonukleotide **GRkurz** und **GR** wurde ein IL-PCR durchgeführt und die Amplifikate auf PAA-Gelen aufgetrennt. Die reproduzierbare Amplifikation eines für Mycoplasmen typischen IL-PCR Produktmusters konnte nachgewiesen werden (Abb. 16).

Durch die obigen Beispiele konnte gezeigt werden, daß die Inter-LINE-PCR eine Methode zur Identifizierung und taxonomischen Charakterisierung von pro- und eurkaryotischen Mikroorganismen auf Gattung-, Art- und Stammebene ist. Diese Methode kann angewandt werden zur medizinischen Diagnostik, beispielsweise zur Identifizierung und Konstruktion von Transformations- bzw. Tumormarkern in der Krebsdiagnostik, sowie zur Identifizierung von in der medizinischen Diagnostik bedeutsamen pathogenen Bakterien und Pilzen (beispeilsweise Candida-Arten). In bevorzugten Ausführungsformen der Erfindung werden die erfindungsgemäß bereitgestellten Oligonukleotidsequenzen zur Diskriminierung von Mitgliedern der Prokarya, Archaea oder Eukarya eingesetzt, wobei einzelne Taxa und Stämme der Prokarya, Archaea oder Eukarya diskriminierbar sind. Beispielsweise können Hefen und Bakterien diskriminiert werden. Die Diskriminierung umfaßt erfindungsgemäß die Identifizierung und/oder die taxonomische Charakterisierung dieser Organismen.

### LITERATURLISTE

Caetano-Anolles et al., Bio/Technology **9**, 553-557 (1991)
Jeffreys et al., Nature **314**, 26-80 (1985),
Jensen et al., Appl.Environ.Microbiol. **59**, 945-952 (1993)
Grimont & Grimont, Ann.Inst.Pasteur/Microbiol. **137B**, 165-175 (1986)
Gurtler et al., J.Gen.Microbiol. **137**, 2673-2679 (1991)
Hattori et al., Nature **321**, 625-628 (1986)
Katzir et al., Proc.Natl.Acad.Sci.USA **82**, 1054-1058 (1985)
Miki et al., Cancer Research **52**, 643-645 (1992)
Munroe et al., Genomics **19**, 506-514 (1994)
Nelson et al., Proc.Natl.Acad.Sci.USA **86**, 6686-6690 (1989)
Ledbetter & Nelson, in: PCR: A Practical Approach, Oxford Univ. Press (1991)
Louws et al., Appl. Environ.Microbiol., **60**, 2286-2295 (1994)
Mathias et al., Science **254**, 1808-1810 (1991)
Richard et al., Mol.Cell.Biol. **14**, 6689-6695 (1994)
Servomaa & Rytömaa, Int.J.Radiat.Biol. **57**,2 331-343 (1990)
Sambrook et al., Molecular cloning, Cold Spring Harbor Laboratory Press (1989)
Ting & Manos, in: PCR Protocols, Academic Press, Inc., 356-367 (1990)
Vaneechoutte et al., FEMS Microbiol. Letters **93**, 227-234 (1992),
Versalowic et al., Nucleic Acid Res. **19**, 6823-6831 (1991)
Williams et al., Nucleic Acids Res. **18**, 6531-6535 (1990)
Welsh & McClelland, Nucleic Acids Res. **18**, 7213-7218 (1990)
Zietkiewicz et al., Genomics **20**, 173-183 (1994)

### SEQUENZPROTOKOLL

(1) ALLGEMEINE ANGABEN:
   (i) ANMELDER:
      (A) NAME: GSF Forschungszentrum fuer Umwelt und Gesundheit GmbH
      (B) STRASSE: Ingolstaedter Landstr. 1
      (C) ORT: Oberschleissheim
      (E) LAND: Deutschland
      (F) POSTLEITZAHL: 85764
   (ii) BEZEICHNUNG DER ERFINDUNG: Inter-LINE-PCR
   (iii) ANZAHL DER SEQUENZEN: 7
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (vi) DATEN DER URANMELDUNG:
      (A) ANMELDENUMMER: DE 195 18 769.5
      (B) ANMELDETAG: 22-MAY-1995
(2) ANGABEN ZU SEQ ID NO: 1:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA oligonucleotide, consensus sequence of sequences of several clones"
   (iii) HYPOTHETISCH: NEIN
   (iv) ANTISENSE: NEIN
   (vi) URSPRÜNLICHE HERKUNFT:
      (A) ORGANISMUS: Mesocricetus auratus
      (G) ZELLTYP: Embryofibroblast
   (xi) SEQUENZBESCHREIBUNG: SEQ ID NO: 1:
   GAAGGGTCTT TGCCAAACTC 20
(2) ANGABEN ZU SEQ ID NO: 2:
   (i) SEQUENZKENNZEICHEN:
      (A) LANGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA oligonucleotide, consensus sequence of sequences of several clones"
   (iii) HYPOTHETISCH: NEIN
   (iv) ANTISENSE: NEIN
   (vi) URSPRÜNLICHE HERKUNFT:
      (A) ORGANISMUS: Mesocricetus auratus
      (G) ZELLTYP: Embryofibroblast
   (xi) SEQUENZBESCHREIBUNG: SEQ ID NO: 2:
   GAGTTTGGCA AAGACCCTTC 20
(2) ANGABEN ZU SEQ ID NO: 3:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA oligonucleotide, consensus sequence of sequences of several clones"
   (iii) HYPOTHETISCH: NEIN
   (iv) ANTISENSE: NEIN
   (vi) URSPRÜNLICHE HERKUNFT:
      (A) ORGANISMUS: Mesocricetus auratus
      (G) ZELLTYP: Embryofibroblast
   (xi) SEQUENZBESCHREIBUNG: SEQ ID NO: 3:
   CCATGTCTAT CTCCATGCAC 20
(2) ANGABEN ZU SEQ ID NO: 4:
   (i) SEQUENZKENNZEICHEN:
      (A) LÄNGE: 20 Basenpaare
      (B) ART: Mucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA oligonucleotide, consensus sequence of sequences of several clones"
   (iii) HYPOTHETISCH: NEIN
   (iv) ANTISENSE: NEIN
   (vi) URSPRÜNLICHE HERKUNFT:
      (A) ORGANISMUS: Mesocricetus auratus
      (G) ZELLTYP: Embryofibroblast
   (xi) SEQUENZBESCHREIBUNG: SEQ ID NO: 4:
   GTGCATGGAG ATAGACATGG 20
(2) ANGABEN ZU SEQ ID NO: 5:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA oligonucleotide, consensus sequence of sequences of several clones"
   (iii) HYPOTHETISCH: NEIN
   (iv) ANTISENSE: NEIN
   (vi) URSPRÜNLICHE HERKUNFT:
      (A) ORGANISMUS: Mesocricetus auratus
      (G) ZELLTYP: Embryofibroblast
   (xi) SEQUENZBESCHREIBUNG: SEQ ID NO: 5:
   GGCTGCCTTT ATATGTTACT GGCC                                    24
(2) ANGABEN ZU SEQ ID NO: 6:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA oligonucleotide, consensus sequence of sequences of several clones"
   (iii) HYPOTHETISCH: NEIN
   (iv) ANTISENSE: NEIN
   (vi) URSPRÜNLICHE HERKUNFT:
      (A) ORGANISMUS: Mesocricetus auratus
      (G) ZELLTYP: Embryofibroblast
   (xi) SEQUENZBESCHREIBUNG: SEQ ID NO: 6:
   GGAGTGCTGA CTCATACAGC CTCC                                    24
(2) ANGABEN ZU SEQ ID NO: 7:
   (i) SEQUENZKENNZEICHEN:
      (A) LÄNGE: 23 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA oligonucleotide, consensus sequence of sequences of several clones"
   (iii) HYPOTHETISCH: NEIN
   (iv) ANTISENSE: NEIN
   (vi) URSPRÜNLICHE HERKUNFT:
      (A) ORGANISMUS: Mesocricetus auratus
      (G) ZELLTYP: Embryofibroblast
   (xi) SEQUENEBESCHREIBUNG: SEQ ID NO: 7:

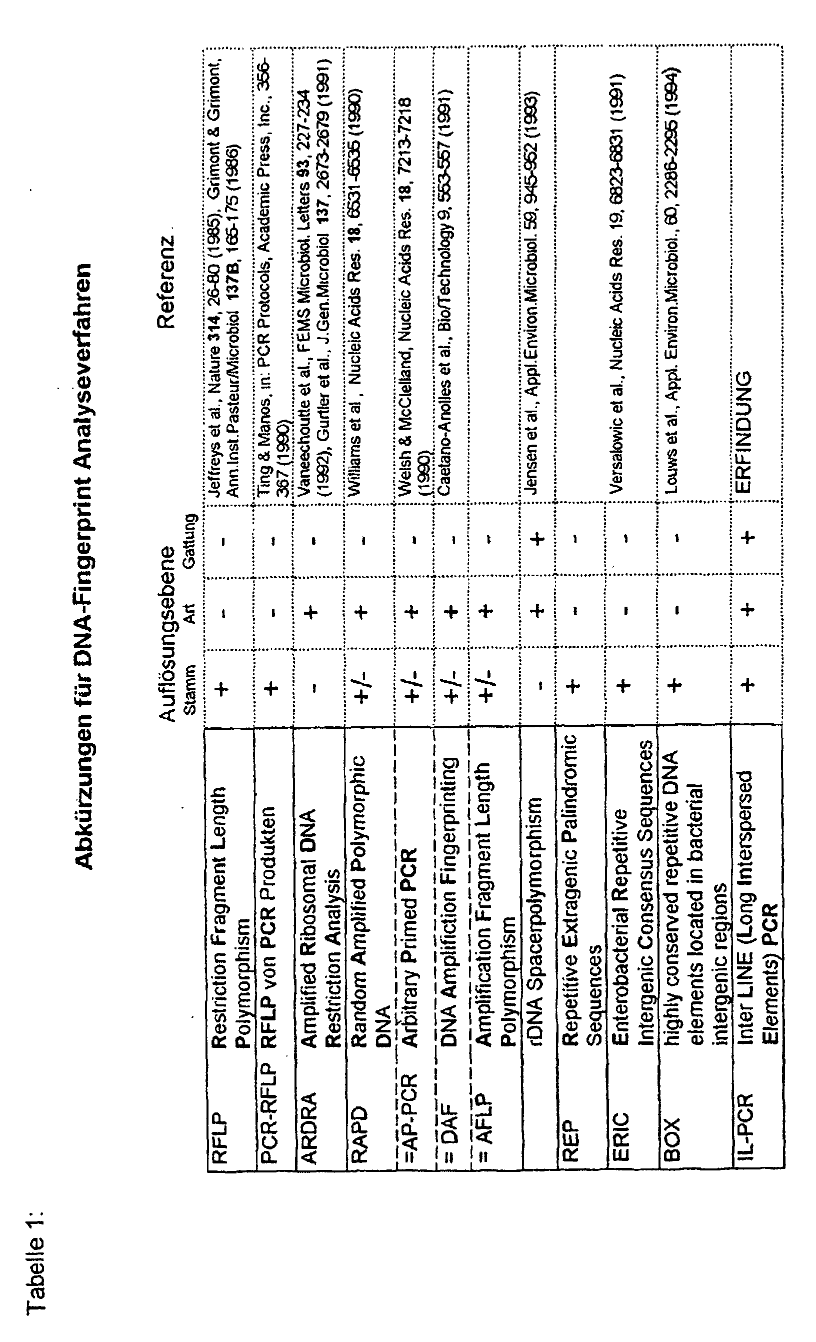

## Patentansprüche

1. Oligonukleotide,
**dadurch gekennzeichnet,**
**daß** sie aus einer oder mehreren der nachfolgenden Oligonukleotide ausgewählt werden: 8. Oligonukleotiden mit einer Homologie von ≥ 95% zu SEQ ID NO:1-7
9. Oligonukleotide, ausgewählt aus den obigen Oligonukleotiden 1-8, wobei am 5'-Ende weitere, zusätzliche Nukleotide angefügt sind.

2. Oligonukleotide,
**dadurch gekennzeichnet,**
**daß** sie aus einer oder mehreren am 3'- und/oder am 5'-Ende verkürzten Oligonukleotiden 1-8 nach Anspruch 1 ausgewählt werden, wobei die verkürzten Oligonukleotide mindestens 15 Nukleotide umfassen, wobei an diese verkürzten Oligonukleotide wahlweise am 5-'Ende weitere, zusätzliche Nukleotide angefügt sind.

3. Oligonukleotide nach Anspruch 1 oder 2,
**dadurch gekennzeichnet,**
**daß** das 5'-Ende modifiziert ist.

4. Oligonukleotide nach Anspruch 3,
**dadurch gekennzeichnet,**
**daß** die Modifikationen am 5'-Ende aus einem oder mehreren der Elemente der Gruppe, bestehend aus Aminosäuren, Digoxigenin-Markierung, Biotin-Markierung ausgewählt werden.

5. Oligonukleotide nach Anspruch 1 oder 2,
**dadurch gekennzeichnet,**
**daß** die zusätzlichen Nukleotide eine oder mehrere Restriktionsenzymerkennungsschnittstellen umfassen.

6. Oligonukleotide nach einem oder mehreren der vorhergehenden Ansprüche,
**dadurch gekennzeichnet,**
**daß** sie in einem Vektorsystem, bevorzugt einem Plasmid, enthalten sind.

7. Verfahren zur Durchführung einer Inter-LINE-PCR,
**gekennzeichnet durch**
die nachfolgenden Verfahrensschritte:
a) Vermischen der zu analysierenden DNA mit einem oder mehreren Oligonukleotiden mit einer Sequenz nach einem oder mehreren der Ansprüche 1 bis 6 in einem zur Durchführung einer PCR-Reaktion geeigneten Reaktionspuffer und wahlweise weiteren Zusätzen;
b) Durchführung von mehrere De- und Renaturierungen umfassenden Amplifikationsschritten zur Gewinnung amplifizierter DNA-Produkte; und
c) Auftrennung und Analyse der Amplifikationsprodukte.

8. Verfahren nach Anspruch 7,
**dadurch gekennzeichnet,**
**daß** die Amplifikationsschritte einen ersten Denaturierungsschritt, einen oder mehrere De- und Renaturierungen mit niedriger Stringenz und mehrere De- und Renaturierungen mit höherer Stringenz und einen abschließenden Kettenverlängerungsschritt umfassen.

9. Verfahren nach Anspruch 8,
**dadurch gekennzeichnet,**
**daß** 3 - 7 De- und Renaturierungsschritte mit niedriger Stringenz und 20 - 30 De- und Renaturierungsschritte mit höherer Stringenz durchgeführt werden.

10. Verwendung eines oder mehrerer Oligonukleotide mit einer Sequenz nach einem oder mehreren der Ansprüche 1 - 6 als Primer für eine IL-PCR.

11. Verwendung nach Anspruch 10 zur in vitro Diskriminierung von DNA der Mitglieder der Prokarya, Archaea oder Eukarya oder genetisch veränderter Organismen hiervon.

12. Verwendung nach Anspruch 10 oder 11 zur in vitro Diskriminierung einzelner Taxa und Stämme der Prokarya, Archaea oder Eukarya.

13. Verwendung nach Anspruch 10 in der medizinischen Diagnostik.

## Claims

1. Oligonucleotides, wherein said oligonucleotides are selected from one or more of the following sequences: 8. Oligonucleotides with a homology of ≥ 95% to SEQ ID-No. 1-7.
9. Oligonucleotides selected from oligonucleotides 1-8 as identified above, wherein further additional nucleotides are added at the 5' end.

2. Oligonucleotides according to claim 1, wherein said oligonucleotides 1-8 are selected from one or more of the sequences shortened at the 3' and/or the 5' terminus of the sequence according to claim 1, the shortened oligonucleotides comprising at least 15 nucleotides, wherein, optionally at the 5' end, further additional nucleotides are added to said shortened oligonucleotides.

3. Oligonucleotides according to claim 1 or 2, wherein the 5' terminus is modified.

4. Oligonucleotides according to claim 3, wherein the modifications at the 5' terminus are selected from one or more members of the group consisting of amino acids, digoxigenin labelling, biotin labelling and further additional nucleotides.

5. Oligonucleotides according to claim 1 or 2, wherein said additional nucleotides comprise one or more restriction sites for restriction enzyme recognition.

6. Oligonucleotides according to one or more of the preceding claims, wherein said oligonucleotides are included in a vector system, preferably a plasmid.

7. Method for performing an Inter-LINE-PCR, comprising the following steps:
(a) mixing in a reaction buffer suitable for carrying out a PCR reaction and optionally further additives of the DNA to be analyzed with one or more oligonucleotides having a sequence according to one or more of claims 1-6,
(b) performing of amplification steps comprising several denaturations and renaturations to obtain amplified DNA products; and
(c) separating and analyzing said amplification products.

8. Method according to claim 7, wherein said amplification steps comprise one initial denaturation step, one or more low stringency denaturations and renaturations and one or more higher stringency denaturations and renaturations, and a final chain elongation step.

9. Method according to claim 8, wherein 3 - 7 low stringency denaturation and renaturation steps and 20 - 30 higher stringency denaturation and renaturation steps are performed.

10. Use of one or more of the oligonucleotides with a sequence in accordance with one or more of claims 1-6 as a primer for an IL-PCR.

11. Use according to claim 10 for the in vitro discrimination of DNA from the members of prokarya, archaea or eukarya or genetically altered organisms thereof.

12. Use in accordance with claim 10 or 11 for the in vitro discrimination of single taxa and phyla of prokarya, archaea or eukarya.

13. Use according to claim 10 in medical diagnostics.

## Revendications

1. Oligonucléotides **caractérisés en ce qu'**ils sont choisis parmi un ou plusieurs des oligonucléotides suivants : 8. oligonucléotides ayant une homologie ≥ 95 % avec SEQ ID n° 1-7
9. oligonucléotides choisis parmi les oligonucléotides 1-8 ci-dessus, d'autres nucléotides supplémentaires étant ajoutés à l'extrémité 5'.

2. Oligonucléotides **caractérisés en ce qu'**ils sont choisis parmi un ou plusieurs oligonucléotides 1-8 raccourcis à l'extrémité 3' et/ou à l'extrémité 5' selon la revendication 1, les oligonucléotides raccourcis comprenant au moins 15 nucléotides, d'autres nucléotides supplémentaires étant éventuellement ajoutés à l'extrémité 5' de ces oligonucléotides raccourcis.

3. Oligonucléotides selon la revendication 1 ou 2, **caractérisés en ce que** l'extrémité 5' est modifiée.

4. Oligonucléotides selon la revendication 3, **caractérisés en ce que** les modifications à l'extrémité 5' sont choisies parmi un ou plusieurs éléments appartenant au groupe constitué par les aminoacides, un marqueur digoxygénine, un marqueur biotine.

5. Oligonucléotides selon la revendication 1 ou 2, **caractérisés en ce que** les nucléotides supplémentaires comprennent un ou plusieurs sites de coupure par reconnaissance par des enzymes de restriction.

6. Oligonucléotides selon une ou plusieurs des revendications précédentes, **caractérisés en ce qu'**ils sont contenus dans un système vecteur, de préférence un plasmide.

7. Procédé pour l'exécution d'une PCR inter-LINE, **caractérisé par**
les étapes de processus suivantes :
a) mélange de l'ADN à analyser avec un ou plusieurs oligonucléotides comportant une séquence selon une ou plusieurs des revendications 1 à 6, dans un tampon de réaction approprié à l'exécution d'une réaction PCR et éventuellement d'autres additifs ;
b) exécution de plusieurs étapes d'amplification comprenant des dénaturations et renaturations, pour l'obtention de produits de type ADN amplifiés ; et
c) séparation et analyse des produits d'amplification.

8. Procédé selon la revendication 7, **caractérisé en ce que** les étapes d'amplification comprennent une première étape de dénaturation, une ou plusieurs dénaturations et renaturations à faible stringence et plusieurs dénaturations et renaturations à plus forte stringence et une étape finale de prolongement de chaîne.

9. Procédé selon la revendication 8, **caractérisé en ce qu'**on effectue 3-7 étapes de dénaturation et renaturation à faible stringence et 20-30 étapes de dénaturation et renaturation à plus forte stringence.

10. Utilisation d'un ou plusieurs oligonucléotides ayant une séquence selon une ou plusieurs des revendications 1 à 6, en tant qu'amorce pour une IL-PCR.

11. Utilisation selon la revendication 10, pour la discrimination in vitro d'ADN des membres des procaryotes, archéobactéries ou eucaryotes, ou d'organismes génétiquement modifiés appartenant à ces groupes.

12. Utilisation selon la revendication 10 ou 11, pour la discrimination in vitro de taxons individuels et de souches individuelles des procaryotes, archéobactéries ou eucaryotes.

13. Utilisation selon la revendication 10, en diagnostic médical.
